**Europäisches Patentamt**

**European Patent Office**

**Office européen des brevets**

(19)

(11) Veröffentlichungsnummer : **0 049 877**
**B1**

(12) # EUROPÄISCHE PATENTSCHRIFT

(45) Veröffentlichungstag der Patentschrift :
23.05.84

(51) Int. Cl.³ : **C 12 Q    1/56**

(21) Anmeldenummer : **81108116.5**

(22) Anmeldetag : **08.10.81**

(54) Verfahren und Reagenz zur Bestimmung von Antithrombin-BM.

(30) Priorität : 09.10.80 DE 3038163
04.08.81 DE 3050268

(43) Veröffentlichungstag der Anmeldung :
21.04.82 Patentblatt 82/16

(45) Bekanntmachung des Hinweises auf die Patenterteilung : 23.05.84 Patentblatt 84/21

(84) Benannte Vertragsstaaten :
AT BE CH DE FR GB IT LI LU NL SE

(56) Entgegenhaltungen :
EP-A- 0 004 271
FR-A- 2 338 496
CHEMICAL ABSTRACTS, Band 93, Nr. 3, 21. Juli 1980,
Seite 236, Nr. 21515g Columbus, Ohio, U.S.A. K.
BARTL et al.: "A versatile assay method of antithrombin III using thrombin and Tos-Gly-Arg-pNA"
CHEMICAL ABSTRACTS, Band 90, Nr. 21, 21. Mai
1979, Seite 355, Nr. 165628h Columbus, Ohio, U.S.A.
G. KINDNESS et al.: "Potentiation of antithrombin III
activity by dextran sulfate"

(73) Patentinhaber : BOEHRINGER MANNHEIM GMBH
Patentabteilung, Abt. E Sandhofer Strasse 112-132
Postfach 31 01 20
D-6800 Mannheim 31 Waldhof (DE)

(72) Erfinder : Schrenk, Jürgen, Dr. rer. nat.
Kiefernstrasse 4
D-8120 Weilheim (DE)
Erfinder : Wunderwald, Peter, Dr. rer. nat.
Schulstrasse 6
D-8121 Haunshofen (DE)
Erfinder : Lill, Helmut, Dr.
Zugspitzstrasse 24
D-8121 Wielenbach (DE)

(74) Vertreter : Weickmann, Heinrich, Dipl.-Ing. et al
Patentanwälte Dipl.-Ing. H. Weickmann Dipl.-Phys.Dr.
K. Fincke Dipl.-Ing. F.A. Weickmann Dipl.-Chem. B.
Huber Dr.-Ing. H. Liska Möhlstrasse 22
D-8000 München 86 (DE)

**Beschreibung**

Die Erfindung betrifft ein Verfahren und ein Reagenz zur Bestimmung eines neuen Thrombininhibitors, der als Antithrombin BM bezeichnet wird und ein Reagenz zu seiner Durchführung.

Das proteolytische Enzym Thrombin spielt im Blutgerinnungssystem eine wichtige Rolle. Bekannt ist, daß im Gerinnungssystem weiter ein spezifischer Inaktivator für das Thrombin vorkommt, welcher in Gegenwart von Heparin Thrombin zu hemmen vermag und als Antithrombin III bezeichnet wird. Die Verwendung von Heparin als Antikoagulans spielt daher in der Therapie und Forschung eine wichtige Rolle. Desgleichen ist AT III, welches auch als Heparin-Cofaktor bezeichnet wird, ein wichtiger Bestandteil von Testsystemen für die Gerinnungseigenschaften des Plasma. AT III inhibiert jedoch Thrombin auch in Abwesenheit von Heparin in einer zeitabhängigen Reaktion und hemmt außer Thrombin z. B. auch Trypsin, Plasmin und Faktor Xa ist also relativ unspezifisch.

Nunmehr wurde überraschenderweise ein neuer spezifischer Thrombininhibitor gefunden, der sich in vieler Hinsicht von AT III unterscheidet. Er ist in der deutschen Patentschrift (Patentanmeldung) DE-A-30 38 163 näher beschrieben.

Der neue Thrombininhibitor hemmt Thrombin in Gegenwart von Dextransulfat mindestens doppelt so stark wie in Gegenwart von Heparin, Plasmin und Trypsin dagegen praktisch gar nicht, und unterscheidet sich von Antithrombin III immunologisch.

Da der neue Thrombininhibitor weniger stark an Heparin bindet als AT III, wird er als AT-BM (Antithrombin Binding Moderately to Heparin) bezeichnet.

Während AT III zur optimalen Thrombinhemmung nur eine Heparinkonzentration in der Größenordnung von 0,02 USP/ml benötigt, liegt bei AT-BM die erforderliche Heparinkonzentration zur optimalen Thrombinhemmung über 1,0 USP/ml.

Während Human-AT-III unter anderem auch Faktor Xa, Plasmin und Trypsin hemmt und Trypsin hemmt und mit Humanthrombin unwesentlich stärker als mit Rinderthrombin reagiert, ist das neue Human-AT-BM nahezu vollständig thrombinspezifisch und inhibiert Humanthrombin deutlich stärker als Rinderthrombin. Ferner wird die Thrombinhemmung durch AT III durch Heparin stärker gefördert als durch andere Aktivatoren, wie Dextransulfat. Dagegen wird bei AT-BM mit Dextransulfat eine 2- bis 3-mal so starke Thrombinhemmung erzielt als in Gegenwart von Heparin.

Während AT III Thrombin in einer zeitabhängigen Reaktion auch in vollständiger Abwesenheit von Co-Faktoren, wie Heparin hemmt, zeigt AT-BM unter diesen Umständen keinerlei Hemmwirkung.

Aufgrund der obigen Eigenschaften kann der erfindungsgemäße neue Thrombininhibitor AT-BM nicht nur ähnlich wie AT III bei therapeutischen und diagnostischen Verfahren eingesetzt werden und stellt eine interessante Forschungschemikalie dar, sondern läßt sich in Gegenwart von AT III auch analytisch bestimmen.

Erfindungsgemäß erfolgt daher die Bestimmung von AT-BM durch Ausnutzung der Spezifitätsunterschiede gegenüber AT III bei den verschiedenen Cofaktoren. Das erfindungsgemäße Verfahren zur Bestimmung von AT-BM ist daher dadurch gekennzeichnet, daß man der Probelösung einen Antithrombin-BM-Cofaktor, Thrombin und ein bestimmbares Thrombinsubstrat zusetzt und die Spaltung des Thrombinsubstrats als Maß für die Antithrombin-BM-Aktivität ermittelt, wobei man

a) die Spezifitätsunterschiede von Antithrombin-BM gegenüber Antithrombin-III in Gegenwart unterschiedlicher Mengen der Cofaktoren Heparin, Xylan oder λ-Karrageenan ausnützt und die Differenz der bei hohen und bei niedrigen Cofaktorkonzentrationen erhaltenen Aktivitäten ermittelt oder

b) mit Dextransulfat, Mucopolysaccharid-polyschwefelsäureester, Pentosanpolysulfate oder durch chemische oder enzymatische Spaltung von Heparin hergestellten kurzkettigen Heparinderivaten als Cofaktor direkt die Antithrombin-BM-Aktivität mißt.

Die Spezifitätsunterschiede von AT-BM im Vergleich zu AT III in Gegenwart unterschiedlicher Mengen Heparin zeigt die nachstehende Tabelle 1.

Tabelle 1

Spezifitätsvergleich AT-BM/AT III

| Protease | AT III (U/ml) mit | | AT-BM (U/ml) | |
|---|---|---|---|---|
| | 0,02 USP Heparin/ml Testvolumen | 1,75 USP Heparin/ml Testvolumen | 0,02 USP Heparin/ml Testvolumen | 1,75 USP Heparin/ml Testvolumen |
| Rinderthrombin Gesamt | 22,5 | 24,4 | 6,6 | 29,0 |
| « α-Thrombin » | 25,9 | 25,6 | 3,7 | 40,3 |
| « β-Thrombin » | 20,0 | 23,5 | 0,4 | 4,5 |

Tabelle 1 (Fortsetzung)

Spezifitätsvergleich AT-BM/AT III

| Protease | AT III (U/ml) mit | | AT-BM (U/ml) | |
|---|---|---|---|---|
| | 0,02 | 1,75 | 0,02 | 1,75 |
| | USP Heparin/ml Testvolumen | | USP Heparin/ml Testvolumen | |
| Humanthrombin | | | | |
| Gesamt | 29,4 | 30,8 | 8,4 | 74,0 |
| « α-Thrombin » | 23,0 | 31,7 | 6,2 | 101,1 |
| « β-Thrombin » | 29,0 | 37,7 | 0,1 | 18,9 |
| Faktor Xa | 5,6 | 29,5 | 0 | 0 |
| Plasmin | 0 | 14,5 | 0 | 0,4 |
| Trypsin | 23,5 | 16,5 | 0 | 0 |

Eine bevorzugte Ausführungsform des erfindungsgemäßen Verfahrens besteht daher darin, daß man bei einer Konzentration von 1 bis 20 USP Heparin pro ml die Gesamtmenge von AT-BM und AT III bestimmt und in einem Parallelansatz bei einer niedrigen Cofaktor-Konzentration von 0,01 bis 0,03 USP Heparin pro ml AT III alleine bestimmt und aus der Differenz der beiden Antithrombinmengenbestimmungen den Gehalt an AT-BM ermittelt. Anstelle von Heparin können als Cofaktor auch Xylan oder λ-Karrageenan in analogen Mengen eingesetzt werden.

Bei der zweiten Ausführungsform des erfindungsgemäßen Verfahrens verwendet man als Cofaktoren Dextransulfat, Mucopolysaccharid-polyschwefelsäureester, Pentosanpolysulfate oder ein durch chemische oder enzymatische Spaltung von Heparin hergestelltes kurzkettiges Heparinderivat. Derartige Heparinderivate bzw. Heparinanaloga sind bekannt und im Handel z. B. unter der Bezeichnung Arteparon bzw. SP 54 erhältlich. Bei Konzentrationen zwischen etwa 2,5 und 25 µg/ml dieser Cofaktoren ist lediglisch AT-BM aktiv, so daß hierbei auch in Gegenwart von AT III selektiv der Gehalt an AT-BM erfaßt wird.

Gleichgültig, ob man die Differenzmethode oder die direkte spezifische AT-BM-Bestimmung anwendet, setzt man zweckmäßig für die Bestimmung 0,01 bis 0,1 U/ml Thrombin, 0,165 bis 1 000 µmol/ml Thrombinsubstrat und 10 bis 250 mmol/l Puffer pH 7,5 bis 8,5 ein, wobei man zusätzlich noch bis zu 200 mmol/l NaCl und bis zu 50 IU/ml Aprotinin zusetzen kann. Die beiden letztgenannten Substanzen sind jedoch nicht essentiell und können auch weggelassen werden.

Außerdem erwies sich ein Zusatz an Polyäthylenglykol und/oder eines Komplexbildners für Schwermetalle wie z. B. Äthylendiamintetraessigsäure und ähnliche Polyaminacetate als günstig.

Als Thrombinsubstrat läßt sich jedes der für die Thrombinbestimmung bekannten Substrate verwenden. Bevorzugt werden die handelsüblichen optisch bestimmbaren Substrate. Bei letzteren handelt es sich um niedermolekulare Peptide mit einem optisch leicht bestimmbaren abspaltbaren Rest. Besonders bevorzugt wird als Substrat Tos-Gly-Pro-Arg-pNA.

Als Puffersubstanzen können alle im Bereich von 7,5 bis 8,5 wirksamen verwendet werden. Bevorzugt werden Trispuffer, Triäthanolaminpuffer und Tris-Imidazolpuffer.

Im normalen Humanplasma liegt AT-BM neben AT III vor und liefert im Durchschnitt etwa 20 % der gesamten Antithrombin-Aktivität, wobei die restlichen etwa 80 % dem AT III zukommen. Das Verhältnis von AT-BM zu AT III kann unter bestimmten Umständen verändert sein. Dies zeigen die in Tabelle 2 zusammengefaßten Versuchsergebnisse.

Tabelle 2

Antithrombin-Bestimmung mit Rinderthrombin und 1,75 USP U/ml Heparin bei 25 °C

| Plasma | n | ∅ Gesamt-AT U/ml | % Gesamt-AT | |
|---|---|---|---|---|
| | | | AT-BM | AT III |
| Normal | 5 | 8,4 ± 0,3 | 20,8 ± 5,4 | 79,2 ± 5,4 |
| Schwangere | 3 | 7,5 ± 2,5 | 14,0 ± 2,4 | 86,0 ± 2,4 |
| Kinder | 6 | 6,1 ± 0,4 | 15,5 ± 0,5 | 84,5 ± 0,5 |
| Marcumar | 8 | 5,7 ± 1,4 | 11,4 ± 4,5 | 88,5 ± 3,9 |
| Hepatitis-Verdacht | 3 | 4,6 ± 1,9 | 10,0 ± 5,6 | 90,0 ± 5,6 |
| Serum | 3 | 3,9 ± 0,7 | 32,0 ± 4,4 | 68,0 ± 4,4 |

Wie erwähnt ist auch die Cofaktor-Spezifität des neuen AT-BM verschieden von der von AT III. Insbesondere wird bei AT III die höchste Hemmkapazität in Gegenwart von Heparin erzielt, während bei AT-BM mit Dextransulfat noch wesentlich höhere Hemmwerte erzielbar sind. Die ermittelten Werte zeigt Tabelle 3.

Tabelle 3

Vergleich der Cofaktor-Spezifität von AT III/AT-BM bei hemmung von Rinderthrombin

| Cofaktor (25 μg/ml) | Antithrombinaktivität U/ml von | |
|---|---|---|
| | AT III | AT-BM |
| Heparin (15 μg/ml = 1,75 USP/ml) | 27,5 | 13,2 |
| λ-Karrageenan | 5,4 | 9,3 |
| Xylan | 6,3 | 1,2 |
| Na-Dextransulfat (MG = 500 000) | 4,1 | 33,0 |
| Polystyrolsulfonat | 0 | 0 |

Auch von anderen bekannten Proteaseinhibitoren im Plasma unterscheidet sich AT-BM in seinen Eigenschaften erheblich. Dies geht aus der nachfolgenden Tabelle 4 augenscheinlich hervor.

Tabelle 4

Vergleich von AT-BM mit den bekannten Proteaseinhibitoren im menschlichen Plasma

| Inhibitor | MG in KD (1) | Bindung an Heparin-Seph. | Immunol. Kreuz-reaktion mit AT-BM | Inhibition von | | | | |
|---|---|---|---|---|---|---|---|---|
| | | | | Thrombin | Faktor Xa | Plasmin | Trypsin | Chymo-trypsin |
| α-1-Antitrypsin | 54 | − | − | + | | + | + | + |
| α-1-Antichymo-trypsin | 68 | − | − | | | | | + |
| Inter-α-trypsin-Inhibitor | 140 | + | − | + | | + | ± | ± |
| Säurestabiler Proteinase-inhibitor | 34 | | | | | | | |
| α₂-Makroglobulin | 725 | − | − | + | | + | + | + |
| α₂-Antiplasmin | 70 | − | | | | + | + | |
| Antithrombin III | 67 | ++ | − | ++ | ++ | + | + | |
| C 1-Esterase-Inaktivator I | 105 | ± | − | | | + | − | |
| C 1-Esterase-Inaktivator II | 96 | | | | | + | − | |
| Antithrombin-BM | 68-69 | + | entfällt | ++ | − | − | − | |

(Inter-α-trypsin-Inhibitor und Säurestabiler Proteinase-inhibitor: CRM²; C 1-Esterase-Inaktivator I und II: CRM²)

(1) KD = Kilodalton.
(2) CRM = untereinander immunologisch kreuzreagierend.

Ein weiterer Gegenstand der Erfindung ist ein Reagenz zur Bestimmung von AT-BM. Das erfindungsgemäße Reagenz ist dadurch gekennzeichnet, daß es Thrombin, ein bestimmbares Thrombinsubstrat, Puffer pH 7,5 bis 8,5, gegebenenfalls NaCl oder/und Aprotinin sowie einen der Cofaktoren Heparin, Xylan, λ-Karrageenan, Dextransulfat, Mucopolysaccharidpolyschwefelsäureester, Pentosanpolysulfate oder durch chemische oder enzymatische Spaltung von Heparin hergestelltes kurzkettiges Heparinderivat enthält.

In einer bevorzugten Ausführungsform ist das erfindungsgemäße Reagenz dadurch gekennzeichnet, daß es Thrombin, Heparin, Aprotinin, Polyäthylenglykol, Komplexbildner, NaCl, Thrombinsubstrat und Puffer enthält. Besonders bevorzugte Ausführungsformen sind dadurch charakterisiert, daß es Thrombin, Dextransulfat, Aprotinin, NaCl, Puffer und Thrombinsubstrat enthält ; daß das Thrombinsubstrat aus Tos-Gly-Pro-Arg-pNA besteht ; daß es 0,01 bis 0,1 U/ml Thrombin, 0,165 bis 1 000 μmol/ml Thrombinsubstrat, 0 bis 200 mmol/l NaCl, 0 bis 50 IU/ml Aprotinin und 10 bis 250 mmol/l Puffer enthält.

Die Gewinnung des Thrombininhibitors AT-BM ist im deutschen Patent DE-C-30 38 163 beschrieben.

Sie beruht auf seinen unterschiedlichen Eigenschaften im Vergleich zu AT III, beispielsweise gegenüber Heparin.

Wenn man Serum, Plasma oder eine daraus gewonnene Fraktion mit unlöslichem, trägergebundenem Heparin behandelt, letzteres nach Entfernung des nichtgebundenen Materials mit einer Ionenstärke von 0,12 bis 0,36 und einem pH-Wert im Bereich von 6,5 bis 8,3 eluiert, so gewinnt man das AT-BM. Unter diesen Bedingungen wird AT III nicht eluiert.

Zur Einstellung des pH-Wertbereichs können die in diesem Bereich puffernden Puffersubstanzen verwendet werden. Die Konzentration des Puffers liegt zweckmäßig zwischen etwa 0,005 und 0,1 M. Die besten Ergebnisse werden bei pH-Werten zwischen 7,5 und 8,0 erhalten. Bevorzugt werden Phosphat- und Tris-Puffer.

Zweckmäßig wird in Gegenwart eines Sequestrierungsmittels, wie EDTA, gearbeitet. Die Konzentration desselben liegt zweckmäßig zwischen 5 und 20 mM.

Die folgenden Beispiele erläutern die Erfindung weiter :

## Beispiel 1

9 Teile frisch entnommenes Blut werden mit 1 Teil 0,11 mol/l Natriumcitrat gemischt und bei ca. 3 000 U/min zentrifugiert. 20 µl des so erhaltenen Citratplasmas werden mit 1,0 ml 0,9 %iger NaCl verdünnt.

5 ml einer Lösung, die 0,1 M Tris/HCl, pH 8,1, 0,15 M NaCl, 0,01 M EDTA, 1 % Polyäthylenglykol, 6,5 IU Aprotinin/ml und 1,75 USP Heparin/ml enthält, werden mit 0,25 ml einer Thrombinlösung mit 0,5 U/ml gemischt und 30 Minuten stehengelassen (= AT-Reaktionsgemisch). Dann erfolgt die Bestimmung gemäß nachstehendem Schema bei einer Meßtemperatur von 25 °C, Küvette von 1 cm Schichtdicke und einer Wellenlänge Hg 405 nm. Gemessen wird die Extinktionszunahme gegen Luft.

In Kunststoffküvetten pipettieren

|  | Ausgangswert | Probe |
|---|---|---|
| 0,9 % NaCl | 0,10 ml | — |
| verd. Plasma | — | 0,10 ml |
| AT-Reaktionsgemisch | 2,00 ml | 2,00 ml |

mischen und 5 Minuten bei 25 °C inkubieren

| 1,9 mM Chromozym® TH ($^1$) | 0,20 ml | 0,20 ml |
|---|---|---|

mischen, innerhalb von 30 Sekunden Anfangsextinktion ablesen und gleichzeitig Stoppuhr starten. Nach genau 30, 60 und 90 Sekunden Ablesung wiederholen.

Aus den Extinktionsdifferenzen pro 30 Sekunden ($\Delta$E/30 Sekunden) Mittelwert bilden und diesen in die Berechnung einsetzen.

1) = Tos-Gly-Pro-Arg-pNA

Man erhält so die gesamte Antithrombinaktivität. Durch Wiederholung des Ansatzes mit auf 0,02 USP/ml erniedriger Heparinkonzentration erhält man die AT III-Konzentration. Aus der Differenz Gesamtantithrombinaktivität minus AT III ergibt sich die Menge an AT-BM.

## Beispiel 2

| Reagentien : | Konzentration der gebrauchsfertigen Lösung |
|---|---|
| 1 Puffer | |
| Tris/HCl | 100 mmol/l, pH 8,1 |
| Dextransulfat (MG 500 000) | 1 mg/100 ml |
| Aprotinin | 6,5 IU/ml* |
| NaCl | 150 mmol/l |
| 2 Thrombin | 0,5 U/ml |
| 3 Chromozym® TH (Tos-Gly-Pro-Arg-pNA) | 1,9 mmol/l |
| 4 Mischung aus 1 und 2 | |
| Tris/HCl | 90 mmol/l, pH 8,1 |
| Dextransulfat | 0,9 mg/100 ml |
| Aprotinin | 5,9 IU/ml |
| NaCl | 136 mmol/l |
| Thrombin | 0,045 U/ml |

*Inhibitor-Einheiten (Trypsin, Chromozym TH, 25 °C)

# 0 049 877

Herstellung des Reagenzgemischs (Mischung aus 1 und 2)

In einem Kunststoffgefäß mischen und ca. 30 min. bei 25 °C stehen lassen : 10 ml Puffer (Lösung 1) und 1 ml Thrombin (Lösung 2).

## Probenvorbereitung

Für die Bestimmung ein Teil Citratplasma mit 200 Teilen NaCl (0,9 %ig) verdünnen (z. B. 50 μl Plasma + 10 ml 0,9 %ige NaCl-Lösung).

## Bestimmungsansatz

Wellenlänge : Hg 405 nm
Küvette : Kunststoffküvette, 1 cm Schichtdicke
Meßtemperatur : 25 °C
Messung gegen Luft (Extinktionszunahme)
Zu jeder Meßserie ist mindestens 1 Thrombin-Leerwert (TL) erforderlich.

In Kunststoffküvetten pipettieren

|  | TL | Probe |
|---|---|---|
| NaCl 0,9 % | 0,05 ml | — |
| verd. Plasma | — | 0,05 ml |
| Reagenzgemisch (4) | 1,0 ml | 1,0 ml |
| mischen 5 min. bei 25 °C inkubieren | | |
| Lösung 3 | 0,1 ml | 0,1 ml |
| mischen und $\Delta E$/30 sec. berechnen | | |

Berechnung :

$$\Delta E_{TL}/30 \text{ sec} - \Delta E_{Probe}/30 \text{ sec} = \Delta E_{AT-BM}/30 \text{ sec}$$

$$IU_{AT-BM}/ml = \Delta E_{AT-BM}/30 \text{ sec} \times 951,1$$

Für die Bestimmungen wurden Lösungen von AT-BM bzw. AT III hergestellt, die folgenden Gehalt aufwiesen :

AT III : 12,5 IU/ml 25 °C
AT-BM : 12,1 IU/ml 25 °C.

Diese Konzentration entspricht dem normalen Humanplasmagehalt von AT III.

Um die Spezifität der Bestimmung auch in Gegenwart von AT III zu zeigen, wurde die Bestimmung sowohl mit der AT-BM-Lösung als auch mit einem Gemisch der beiden Lösungen durchgeführt. Hierbei wurde wie folgt vorgegangen :

### 1. Messung von AT-BM

Die AT-BM-Lösung wurde 1 + 100 mit physiologischer Kochsalzlösung verdünnt (0,05 ml Probe + 5,0 ml NaCl-Lösung).

Zur Ermittlung des Meßbereiches wurden von dieser Probenverdünnung weitere Verdünnungen mit physiologischer Kochsalzlösung hergestellt (9 Teile AT-BM + 1 Teil NaCl ; 8 + 2 ; 7 + 3 usw.) bis zu einer Endverdünnung von 1 : 1 000.

Konstante Volumina (0,05 ml) der hergestellten AT-BM-Verdünnungen wurden sodann in den Test eingesetzt.

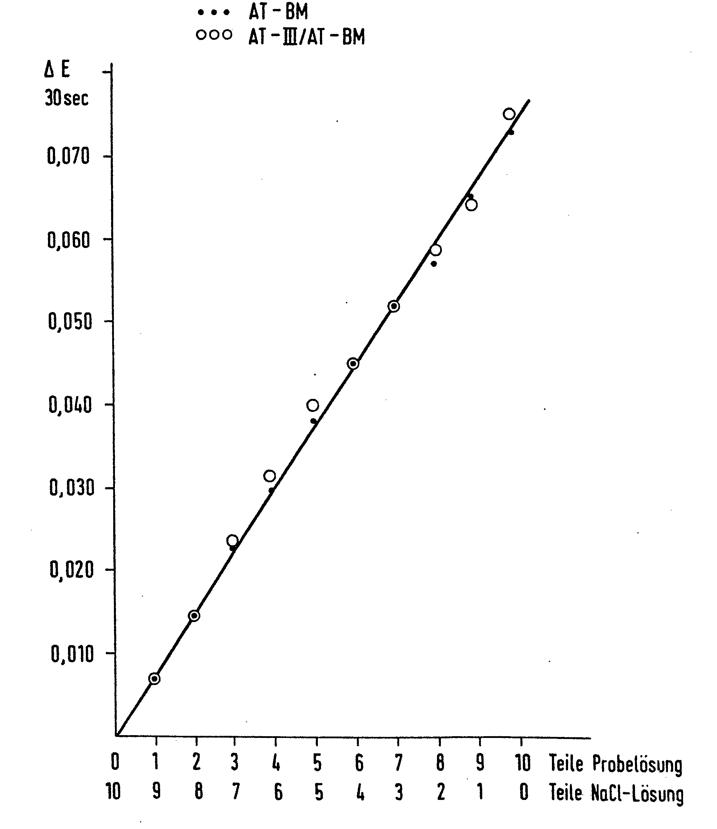

Das Ergebnis ist dargestellt in Fig. 1 der Zeichnung (●●●●). Der Test mißt innerhalb der gewählten Grenzen proportionale Hemmwerte ($\Delta E_{AT-BM}$).

### 2. Messung von AT-BM in Gegenwart von AT-III

0,05 ml AT-BM-Lösung wurden mit 0,05 ml AT-III-Lösung und 4,95 ml physiologischer Kochsalzlösung verdünnt (entsprechend einer 1 + 100 Verdünnung beider Komponenten).

Von dieser Probelösung wurden Folgeverdünnungen hergestellt wie unter 1. beschrieben.

0 049 877

Konstante Volumina (0,05 ml) dieser hergestellten Verdünnungen wurden sodann in den Test eingesetzt.

Das Ergebnis ist dargestellt in Fig. 1 (OOOOOO). Es wird innerhalb der Testgenauigkeit das gleiche Ergebnis erhalten wie mit AT-BM allein. Das Testergebnis wird durch AT-III nicht beeinflußt ; — es wird allein AT-BM gemessen.

Analoge Ergebnisse wurden erhalten, wenn Dextransulfat durch Mucopolysaccharid-polyschwefelsäureester, Pentosanpolysulfate oder durch die oben näher erläuterten Heparinderivate ersetzt wurde.

**Ansprüche**

1. Verfahren zur Bestimmung von Antithrombin-BM, dadurch gekennzeichnet, daß man der Probelösung einen Antithrombin-BM-Cofaktor, Thrombin und ein bestimmbares Thrombinsubstrat zusetzt und die Spaltung des Thrombinsubstrats als Maß für die Antithrombin-BM-Aktivität ermittelt, wobei man

a) die Spezifitätsunterschiede von Antithrombin-BM gegenüber Antithrombin-III in Gegenwart unterschiedlicher Mengen der Cofaktoren Heparin, Xylan oder λ-Karrageenan ausnützt und die Differenz der bei hohen und bei niedrigen Cofaktorkonzentrationen erhaltenen Aktivitäten ermittelt oder

b) mit Dextransulfat Mucopolysaccharid-polyschwefelsäureester, Pentosanpolysulfate oder durch chemische oder enzymatische Spaltung von Heparin hergestellten kurzkettigen Heparinderivaten als Cofaktor direkt die Antithrombin-BM-Aktivität mißt.

2. Verfahren nach Anspruch 1, dadurch gekennzeichnet, daß man als hohe Konzentration 1 bis 20 USP/ml, als niedrige Konzentration 0,01 bis 0,03 USP Heparin pro ml anwendet.

3. Verfahren nach Anspruch 1, dadurch gekennzeichnet, daß man 2,5 bis 25 µg/ml Dextransulfat oder Heparinspaltprodukt einsetzt.

4. Verfahren nach Anspruch 1, 2 oder 3, dadurch gekennzeichnet, daß man 0,01 bis 0,1 U/ml Thrombin, 0,165 bis 1 000 µmol/ml Thrombinsubstrat, 0 bis 200 mmol/l NaCl, 0 bis 50 IU/ml Aprotinin und 10 bis 250 mmol/l Puffer pH 7,5 bis 8,5 einsetzt.

5. Verfahren nach Anspruch 2, dadurch gekennzeichnet, daß man der Lösung Polyäthylenglykol und einen Komplexbildner für Schwermetalle zusetzt.

6. Verfahren nach einem der vorhergehenden Ansprüche, dadurch gekennzeichnet, daß man als Thrombinsubstrat Tos-Gly-Pro-Arg-pNA verwendet.

7. Verfahren nach Anspruch 5, dadurch gekennzeichnet, daß man eine Lösung verwendet, die 0,012 U/ml Thrombin, 6,5 IU Aprotinin pro ml und 1 % Polyäthylenglykol enthält und 0,01 Molar an EDTA, 0,15 Molar an NaCl, 0,1 Molar an Puffer und 0,19 mMolar an Substrat ist.

8. Reagenz zur Bestimmung von Antithrombin-BM, dadurch gekennzeichnet, daß es Thrombin, ein bestimmbares Thrombinsubstrat, Puffer pH 7,5 bis 8,5, gegebenenfalls NaCl oder/und Aprotinin sowie einen der Cofaktoren Heparin, Xylan, λ-Karrageenan, Dextransulfat, Mucopolysaccharid-polyschwefelsäure-re-ester, Pentosanpolysulfate oder durch chemische oder enzymatische Spaltung von Heparin hergestelltes kurzkettiges Heparinderivat enthält.

9. Reagenz nach Anspruch 7, dadurch gekennzeichnet, daß es Thrombin, Heparin, Aprotinin, Polyäthylenglykol, Komplexbildner, NaCl, Thrombinsubstrat und Puffer enthält.

10. Reagenz nach Anspruch 7, dadurch gekennzeichnet, daß es Thrombin, Dextransulfat, Aprotinin, NaCl, Puffer und Thrombinsubstrat enthält.

11. Reagenz nach Anspruch 8 oder 9, dadurch gekennzeichnet, daß das Thrombinsubstrat aus Tos-Gly-Pro-Arg-pNA besteht.

12. Reagenz nach Anspruch 9 oder 10, dadurch gekennzeichnet, daß es 0,01 bis 0,1 U/ml Thrombin, 0,165 bis 1 000 µmol/ml Thrombinsubstrat, 0 bis 200 mmol/l NaCl, 0 bis 50 IU/ml Aprotinin und 10 bis 250 mmol/l Puffer enthält.

**Claims**

1. Process for the determination of antithrombin-BM, characterised in that one first adds to the sample solution an antithrombin-BM cofactor, thrombin and a determinable thrombin substrate and determines the splitting of the thrombin substrate as a measure of the antithrombin-BM activity, whereby one

a) utilises the specificity differences of antithrombin-BM towards antithrombin III in the presence of different amounts of the cofactors heparin, xylan or λ-carrageenan and determines the difference of the activities obtained in the case of high and in the case of low cofactor concentrations or

b) measures the antithrombin-BM activity directly with dextran sulphate, mucopolysaccharide polysulphuric acid esters, pentosan polysulphates or short-chained heparin derivatives produced by chemical or enzymatic splitting of heparin as cofactor.

2. Process according to claim 1, characterised in that as high concentration one uses 1 to 20 USP/ml, and as low concentration 0.01 to 0.03 USP heparin per ml.

7

3. Process according to claim 1, characterised in that one uses 2.5 to 25 µg/ml of extran sulphate or heparin fission product.

4. Process according to claim 1, 2 or 3, characterised in that one uses 0.01 to 0.1 U/ml of thrombin, 165 to 1 000 µmol/ml of thrombin substrate, 0 to 200 mmol/l of NaCl, 0 to 50 IU/ml of aprotonin and 10 to 250 mmol/l of buffer pH 7.5 to 8.5.

5. Process according to claim 2, characterised in that to the solution one adds polyethylene glycol and a complex former for heavy metals.

6. Process according to one of the preceding claims, characterised in that as thrombin substrate one uses Tos-Gly-Pro-Arg-pNA.

7. Process according to claim 5, characterised in that one uses a solution which contains 0.012 U/ml of thrombin, 6.5 IU of aprotonin per ml and 1 % polyethylene glycol and is 0.01 molar for EDTA, 0.15 for NaCl, 0.1 molar for buffer and 0.19 molar for substrate.

8. Reagent for the determination of antithrombin-BM, characterised in that it contains thrombin, a determinable thrombin substrate, buffer pH 7.5 to 8.5, optionally NaCl and/or aprotonin, as well as one of the cofactors heparin, xylan, λ-carrageenan, dextran sulphate, mucopolysaccharide polysulphuric acid esters, pentosan polysulphates or a short-chained heparin derivative produced by chemical or enzymatic splitting of heparin.

9. Reagent according to claim 7, characterised in that it contains thrombin, heparin, aprotonin, polyethylene glycol, complex former, NaCl, thrombin substrate and buffer.

10. Reagent according to claim 7, characterised in that it contains thrombin, dextran sulphate, aprotonin, NaCl, buffer and thrombin substrate.

11. Reagent according to claim 8 or 9, characterised in that the thrombin substrate consists of Tos-Gly-Pro-Arg-pNA.

12. Reagent according to claim 9 or 10, characterised in that it contains 0.01 to 0.1 U/ml of thrombin, 165 to 1,000 µmol/ml of thrombin substrate, 0 to 200 mmol/l of NaCl, 0 to 50 IU/ml of aprotonin and 10 to 250 mmol/l of buffer.

**Revendications**

1. Procédé pour la détermination de l'antithrombine-BM caractérisé en ce que l'on ajoute à la solution de l'échantillon un co-facteur de l'antithrombine-BM, de la thrombine et un substrat de la thrombine apte à une mesure, et on prend la scission du substrat de thrombine en tant que mesure de l'activité de l'antithrombine-BM,

a) en exploitant les différences de spécificité de l'antithrombine-BH comparativement à l'antithrombine-III en présence de quantités différentes des co-facteurs héparine, xylane ou λ-carraghee-nane et en déterminant la différence des activités obtenues aux fortes concentrations et aux basses concentrations en co-facteurs, ou bien

b) en mesurant directement l'activité de l'antithrombine-BM avec du sulfate de dextranne, un ester mucopolysaccharide-polysulfurique, des polysulfates de pentosane ou des dérivés à chaîne courte de l'héparine préparés par scission chimique ou enzymatique de l'héparine, en tant que co-facteur.

2. Procédé selon la revendication 1, caractérisé en ce que l'on utilise en tant que forte concentration 1 à 20 USP/ml et en tant que basse concentration 0,01 à 0,03 USP d'héparine par ml.

3. Procédé selon la revendication 1, caractérisé en ce que l'on utilise 2,5 à 25 µg/ml de sulfate de dextranne ou de produit de scission de l'héparine.

4. Procédé selon la revendication 1, 2 ou 3, caractérisé en ce que l'on utilise 0,01 à 0,1 U/ml de thrombine, 0,165 à 1,000 µmoles/ml de substrat de thrombine, 0 à 200 mmoles/l de NaCl, 0 à 50 UI/ml d'aprotinine et 10 à 250 mmoles/l de tampon pH 7,5 à 8,5.

5. Procédé selon la revendication 2, caractérisé en ce que l'on ajoute à la solution du polyéthylène-glycol et un complexant des métaux lourds.

6. Procédé selon l'une des revendications qui précèdent, caractérisé en ce que l'on utilise en tant que substrat de thrombine Tos-Gly-Pro-Arg-pNA.

7. Procédé selon la revendication 5, caractérisé en ce que l'on utilise une solution qui contient 0,012 U/ml de thrombine, 6,5 UI d'aprotinine par ml et 1 % de polyéthylène-glycol, et est 0,01 molaire en EDTA, 0,15 molaire en NaCl, 0,1 molaire en tampon et 0,19 millimolaire en substrat.

8. Réactif pour la détermination de l'antithrombine-BM, caractérisé en ce qu'il contient de la thrombine, un substrat de la thrombine apte à une détermination, du tampon pH 7,5 à 8,5, le cas échéant du NaCl et/ou de l'aprotinine ainsi que l'un des co-facteurs héparine, xylane, λ-carrageenane, sulfate de dextranne, ester mucopolysaccharide-polysulfurique, polysulfates de pentosane ou dérivé à chaîne courte de l'héparine préparé par scission chimique ou enzymatique de l'héparine.

9. Réactif selon la revendication 7, caractérisé en ce qu'il contient de la thrombine, de l'héparine, de l'aprotinine, du polyéthylène-glycol, du complexant, du NaCl, du substrat de thrombine et du tampon.

10. Réactif selon la revendication 7, caractérisé en ce qu'il contient de la thrombine, du sulfate de dextranne, de l'aprotinine, du NaCl, du tampon et du substrat de thrombine.

11. Réactif selon la revendication 8 ou 9, caractérisé en ce que le substrat de thrombine consiste en Tos-Gly-Pro-Arg-pNA.

12. Réactif selon la revendication 9 ou 10, caractérisé en ce qu'il contient 0,01 à 0,1 U/ml de thrombine, 0,165 à 1 000 μmoles/ml de substrat de thrombine, 0 à 200 mmoles/l de NaCl, 0 à 50 UI/ml d'aprotinine et 10 à 250 mmoles/l de tampon.